(19) Europäisches Patentamt
European Patent Office
Office européen des brevets

(11) **EP 1 075 860 A1**

(12) **EUROPEAN PATENT APPLICATION**
published in accordance with Art. 158(3) EPC

(43) Date of publication:
**14.02.2001 Bulletin 2001/07**

(51) Int. Cl.⁷: **A63B 23/10**, A63B 23/035,
A63B 71/06, A61B 5/05

(21) Application number: **99917189.5**

(22) Date of filing: **27.04.1999**

(86) International application number:
**PCT/JP99/02248**

(87) International publication number:
**WO 99/56834 (11.11.1999 Gazette 1999/45)**

(84) Designated Contracting States:
**AT BE CH CY DE DK ES FI FR GB GR IE IT LI LU MC NL PT SE**

(30) Priority: **01.05.1998 JP 12204598**

(71) Applicant: **Yaman Ltd.**
**Tokyo 135-0045 (JP)**

(72) Inventors:
 • **YAMAZAKI, Iwao**
**Yaman Ltd.**
**Koto-ku, Tokyo 135-0045 (JP)**

• **YAMAZAKI, Kimiyo**
**Yaman Ltd.**
**Koto-ku, Tokyo 135-0045 (JP)**

(74) Representative:
**Newstead, Michael John et al**
**Page Hargrave**
**Southgate**
**Whitefriars**
**Lewins Mead**
**Bristol BS1 2NT (GB)**

(54) **DEVICE FOR DISPLAYING EFFECT OF TRAINING USING WEIGHT MACHINE**

(57)     Disclosed is a muscle strengthening exercise machine equipped with an exercise effect display for showing a time-sequential graph representing how the muscle of a selected part of one's body has been developed with time.

It comprises bodily impedance measuring means 11 having grip-shaped electrodes H to be applied to selected parts of one's body for determining the bodily impedance; data inputting means 12 for inputting individual data pertaining to sex, age, height and weight with the aid of a colour liquid crystal panel; means 13 for determining a bodily fat rate from the bodily impedance and the individual data; means 14 for calculating the non-fat texture weight from the bodily fat rate and the bodily weight; memory means 15 for storing the non-fat texture weight for each measurement; and display means 16 for providing a time-sequential display of so stored non-fat texture weight, thereby showing how the non-fat texture weight varies with time as a consequence of the muscle strengthening exercise.

F I G. 4

EP 1 075 860 A1

## Description

### Technical Field

The present invention relates to aerobic exercise machines particularly intended to strengthen selected muscles of one's body such as arms, breast, abdomen, waist, thigh and calves by loading these parts with a controlled amount of weight.

### Background Art

Treadmills for walking or jogging, bicycle ergometers for pedalling, stair climbers for raising bodily weights, rowing machines and other exercise machines are used in aerobic exercises for strengthening the heart and lung.

Muscles are tightened to produce a variety of movements in daily life. To enjoy an active life while living long it is important to develop one's bodily muscles by using exercise machines designed for that purpose or by taking dumbbell exercises.

Such exercise machines are of weight-stack type, hydrostatic type, pneumatic type or electromagnetic type. The weight-stack type of machine uses pulleys or levers to raise weights. The other types of machines make one to be loaded with a controlled resistance caused by hydrostatic, pneumatic or electromagnetic functions.

If one's muscles are not used, the muscles are prone to atrophy, allowing one's activity to be accordingly lowered. The muscles developed by training are liable to atrophy unless the training is continued. The physical effect caused by a short-term resistance training is mostly attributable to neural effect. The perpetual muscled body can be built only by a long-term workout.

It is important that the workout is so continued as to cause the physical effect evenly on the whole body, not to be localized. Such whole body exercise is effective to prevent the orthopaedic hamper such as pull or distortion, which would be likely to be caused if the opponent or opposite muscles are imbalanced.

It is better to begin with muscle strengthening exercises for developing basal biceps, particularly the major muscle group, which can produce a strong power in activity.

Exercise machines appropriate for the purpose are designed to load one's body repeatedly with a controlled amount of weight for developing the major muscle group evenly in the whole body, so that the whole muscle may be developed under the influence of biological effect and that the muscular function may be improved through the agency of neural activation. Development of bone-surrounding muscles causes the bones to be strengthened, and accordingly the bodily weight, particularly the non-fat texture weight increases.

Non-fat texture weight, therefore, can be a measure representing the physical effect caused by a long-term, muscle-strengthening exercise. Assuming that the muscle development workout is continued a relatively long time of period, such workout can be effectively carried out if one is being informed of increase of non-fat texture weight all the time.

Conventional muscle strengthening exercise machines, however, have no exercise effect display equipped therewith. Therefore, one cannot realize how much one's muscles have been developed, and is apt to discontinue the bodily exercise on the way to the goal.

In view of the above one object of the present invention is to provide a display which is capable of showing a time-sequential change of non-fat texture weight caused by the long-term continuance of muscle strengthening exercise.

### Disclosure of Invention

To attain this object an exercise effect display for a muscle strengthening exercise machine intended to allow one to use any selected part of one's body for strengthening the muscle of the selected part comprises: bodily impedance measuring means having electrodes to be applied to selected parts of one's body for determining the bodily impedance: data inputting means for inputting individual data pertaining to sex, age, height and weight; means for determining a bodily fat rate from the bodily impedance and the individual data; means for calculating the non-fat texture weight from the bodily fat rate and the bodily weight; memory means for storing the non-fat texture weight for each measurement; and display means for providing a time-sequential display of so stored non-fat texture weight, thereby showing how the non-fat texture weight varies with time as a consequence of the muscle strengthening exercise.

**Brief Description of Drawings**

Fig.1 shows diagrammatically an arm-curl machine;
Fig.2 shows diagrammatically a leg-curl machine;
Fig.3 shows diagrammatically an abdominal muscle developing machine;
Fig.4 is a block diagram of the exercise effect display according to the present invention;
Fig.5 shows diagrammatically a pair of handholds in the exercise display;
Fig.6 is a circuit diagram of the bodily impedance measuring circuit; and
Fig.7 shows how a time-sequential display of non-fat texture weight is given on the display

**Best Mode of Reducing the Invention into Practice**

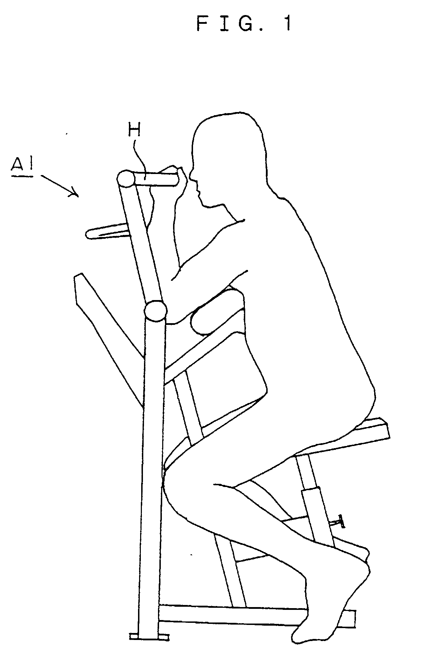

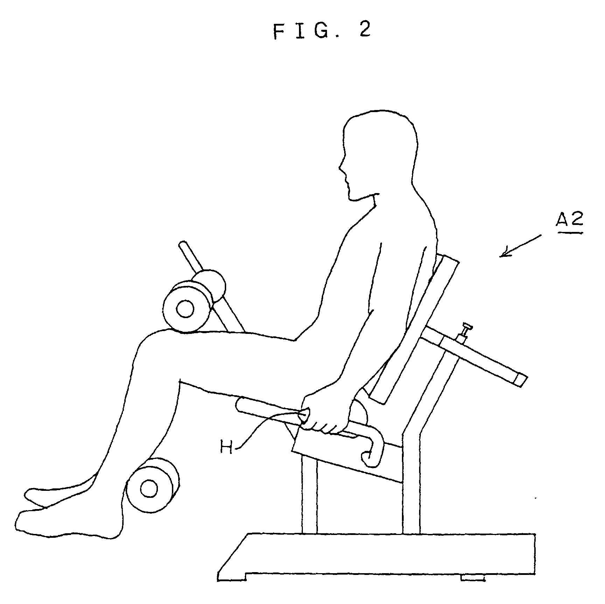

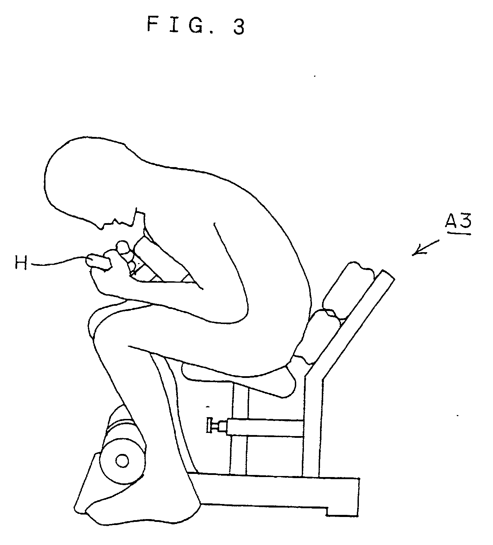

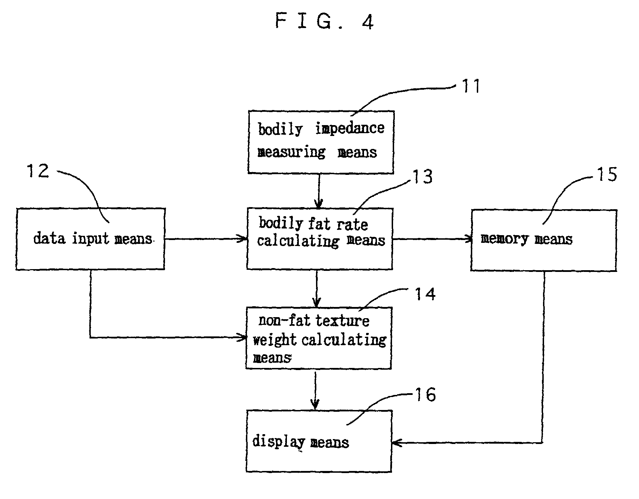

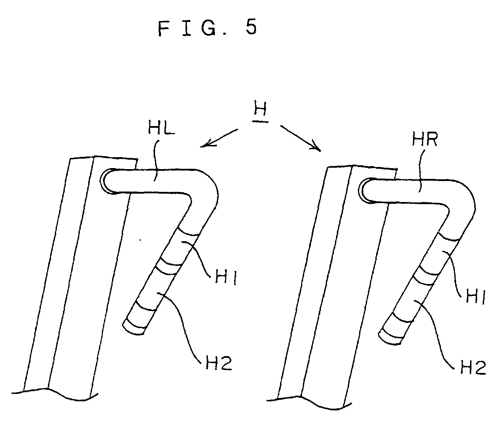

Figs.1 to 3 show diagrammatically different types of muscle strengthening exercise machines which can be equipped with an exercise effect display according to the present invention.
Referring to Fig.1, an arm muscle developing machine A1 is used to repeatedly twist one's upper arms against his resistance, thereby strengthening the biceps of the upper arms.
Referring to Fig.2, a leg muscle developing machine A2 is used to repeatedly twist one's thighs against his resistance, thereby strengthening the hamstring and other muscles.
Referring to Fig.3, an abdominal muscle developing machine is used to repeatedly fold and unfold one's abdomen, thereby strengthening the abdominal muscles.
These muscle strengthening exercise machines are equipped with colour liquid crystal panels, which are responsive to finger touch for showing the loading condition and other exercise or physical data. Measurements of bodily impedance can be effected by holding the handholds H with hands.
Referring to Fig.4, a muscle strengthening exercise effect display 1 according to the present invention comprises bodily impedance measuring means 11 for determining a bodily impedance with the aid of the electrodes of the handholds H, data input means 12 for inputting individual data pertaining to sex, age, height and weight with the aid of the colour liquid crystal display, bodily fat rate calculating means 13 for determining a bodily fat rate both from the bodily impedance and the individual data, non-fat texture weight calculating means 14 for determining non-fat texture weight both from the bodily fat rate and the bodily weight, memory means 15 for storing non-fat texture weights for every measurement, and display means 16 for providing a time-sequential graph of non-fat texture weight. While taking a bodily exercise one's weight can be determined by using a strain gauge, which may be installed in the exercise machine.
Referring to Fig.5, each handhold HL or HR has a feeding electrode H1 and a detecting electrode H2 wound around its insulating rod. The feeding electrode H1 is separated from the counter detecting electrode H2.

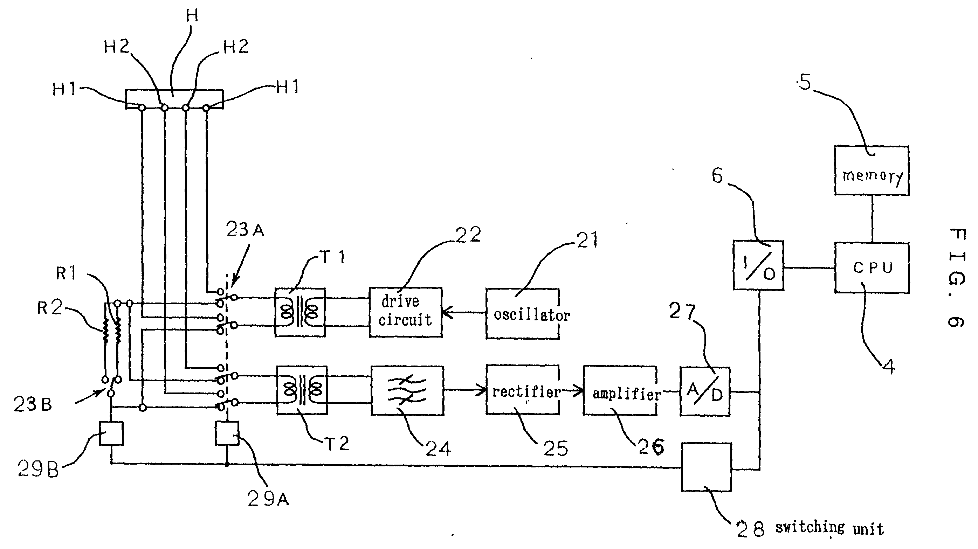

Fig.6 is a block diagram of a measuring circuit of the bodily impedance measuring means 11. As shown in the drawing, an oscillator 21 generates sinusoidal voltage at 50 kHz, which is fed to the feeding electrodes H1 and H1 via a drive circuit 22, a transformer T1 and a switch 23A. When one holds the left and right feeding electrodes H1 and H1 in hands, an ac voltage appears between the detecting electrodes H2 and H2. The ac voltage is converted to a corresponding dc voltage after passing through the switch 23A, a transformer T2, a band filter 24, a rectifier 25 and an amplifier 26, and then, the dc voltage is directed to a CPU 4 via an A/D converter 27 and an I/O interface 6 after being shaped, level-controlled and offset-adjusted. The CPU 4 has a memory 5 associated therewith.
In an attempt to reduce a measurement error which would be caused by the time variability and temperature characteristics of some circuit components the measuring circuit must be calibrated in terms of its input-to-output characteristics prior to measurement of bodily impedance. Specifically the calibration can be effected by using the following equation:

$$Z = kV + C_0$$

where Z stands for bodily impedance; V stands for the voltage appearing between the detecting electrodes H2 and H2; k stands for a proportionality factor; and $C_0$ stands for a fixed constant.
The same ac voltage as used in measuring a bothly impedance Z is applied across two resistances R1 and R2 of known values alternately to determine the voltages appearing across these known resistances R1 and R2. By substituting the known values of R1 and R2 and the so determined voltages for Z and V in two equations two contacts k and $C_0$ can be determined.
To do this the CPU 4 directs a control signal to the switching control 29A via the I/O interface 6 and a switching unit 28 to put the resistance R1 in the measuring circuit. Then, the CPU 4 directs another control signal to the switching control 29R via the I/O interface 6 and the switching unit 28 to put the resistance R2 in the measuring circuit.

The bodily impedance measuring means 11 can determine a bodily impedance according to the following equation 1:

Bodily Impedance = A x Gradient of the Curve x Measured Value + Ordinates Intersection of the Curve

| A = 0.792 | male |
|-----------|--------|
| 0.825 | female |

Gradient = (810-310)/(Upper Measured Value - Lower Measured Value)

Intersection = 810 - Gradient x Upper Measured Value

The bodily fat rate measuring means 13 can determine a bodily fat rate according to the following equation 2:

Females:

Bodily Fat Rate = (1 - Non-Fat Texture/Weight)x100

Non-Fat Texture = 0.6483 x (Height x Height)/Impedance + B x Weight + 5.091

| B = 0.1699 | for Height: 150cm or taller | |
|-----------|-----------------------------|---|
| 0.12 | for Height: below 150cm | Upper Limit of Ideal Weight + 2.5 or more |
| 0.13 | | Upper Limit of Ideal Weight + 2.0 or more |
| 0.14 | | Upper Limit of Ideal Weight + 1.5 or more |
| 0.15 | | Upper Limit of Ideal Weight + 1.0 or more |
| 0.16 | | above Upper Limit of Ideal Weight |
| 0.1699 | | below Upper Limit of Ideal Weight |

Upper Limit of Ideal Weight = (Height - 100) x 0.9 x 1.1

Males:

Bodily Fat Rate = (4.95/Bodily Density - 4.5) x 100

Bodily Density = 1.1554-(0.0841 x Weight x Impedance)/(Height x Height)

The non-fat texture weight calculating means 14 can determine a non-fat texture weight according to the following equation 3:

Non-fat texture weight = bodily weight x (1 - bodily fat rate/100)

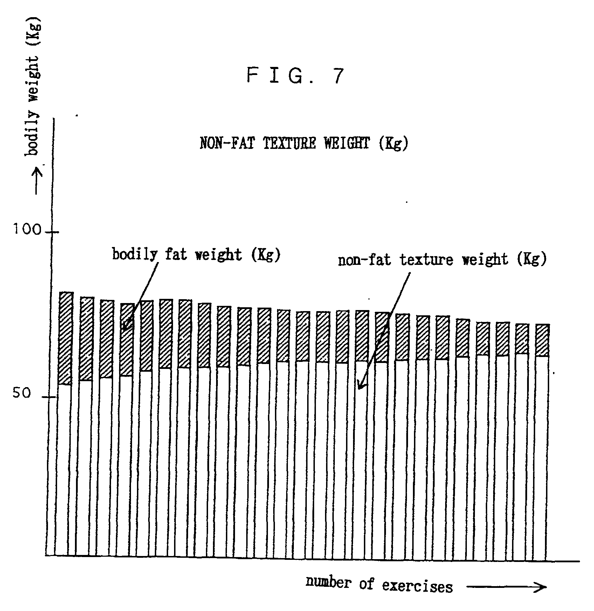

Referring to Fig.7, the display means 16 shows a time-sequential graph of non-fat texture weight on the colour liquid crystal panel. The bar graph has the bodily fat weight (kg) and the non-fat texture weight (kg) on its ordinates, and the number of exercises on its abscissa.

**Possibility of Industrial Utility**

As may be understood from the above, a muscle strengthening exercise effect display according to the present invention determines a bodily impedance from the personal data, that is, sex, age, height and weight to calculate a bodily fat weight from the so determined bodily impedance and a non-fat texture weight from the bodily fat weight. Non-fat texture weights thus determined are stored for every measurement to provide a time-sequential graph of non-fat texture weight on the display.

Thus, one can visually realize the effect of continuing such a muscle strengthening exercise in terms of how the non-fat texture weight representative of growth of bones and muscle has varied, so that he may be encouraged at the result attained. Thus, he can continue the exercise until he has attained his purpose.

Also advantageously, the visual realization of the increase of non-fat texture weight will make him increasingly eager to take such an exercise. As a result his non-fat texture weight can be increased significantly in a short length of time.

**Claims**

1. An exercise effect display for a muscle strengthening exercise machine intended to allow one to use any selected part of one's body for strengthening the muscle of the selected part comprising:

   bodily impedance measuring means having electrodes to be applied to selected parts of one's body for determining the bodily impedance;
   data inputting means for inputting individual data pertaining to sex, age, height and weight;
   means for determining a bodily fat rate from the bodily impedance and the individual data;
   means for calculating the non-fat texture weight from the bodily fat rate and the bodily weight;
   memory means for storing the non-fat texture weight for each measurement; and
   display means for providing a time-sequential display of so stored non-fat texture weight, thereby showing how the non-fat texture weight varies with time as a consequence of the muscle strengthening exercise.

F I G. 1

FIG. 2

FIG. 3

# F I G. 4

bodily impedance
measuring means  — 11

data input means — 12

bodily fat rate
calculating means — 13

memory means — 15

non-fat texture
weight calculating
means — 14

display means — 16

F I G . 5

FIG. 6

# F I G. 7

NON-FAT TEXTURE WEIGHT (Kg)

bodily fat weight (Kg)  non-fat texture weight (Kg)

bodily weight (Kg)

100

50

number of exercises ⟶

# INTERNATIONAL SEARCH REPORT

| International application No. |
|---|
| PCT/JP99/02248 |

**A. CLASSIFICATION OF SUBJECT MATTER**
Int.Cl⁶ A63B23/10, 23/035, 71/06, A61B5/05

According to International Patent Classification (IPC) or to both national classification and IPC

**B. FIELDS SEARCHED**

Minimum documentation searched (classification system followed by classification symbols)
Int.Cl⁶ A63B23/10, 23/035, 71/06, A61B5/05

Documentation searched other than minimum documentation to the extent that such documents are included in the fields searched

| | | | | |
|---|---|---|---|---|
| Jitsuyo Shinan Koho | 1922–1996 | Toroku Jitsuyo Shinan Koho | 1994–1999 | |
| Kokai Jitsuyo Shinan Koho | 1971–1999 | Jitsuyo Shinan Toroku Koho | 1996–1999 | |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, 6-36839, Y2 (YAMAN Ltd.),<br>28 September, 1994 (28. 09. 94),<br>Full text ; Figs. 1 to 5 (Family: none) | 1 |
| Y | JP, 8-126632, A (Omron Corp.),<br>21 May, 1996 (21. 05. 96),<br>Full text ; Figs. 1 to 12 (Family: none) | 1 |
| Y | JP, 8-280840, A (Matsushita Electric Works,Ltd.),<br>29 October, 1996 (29. 10. 96),<br>Full text ; Figs. 1 to 9 (Family: none) | 1 |

☐ Further documents are listed in the continuation of Box C.    ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>22 July, 1999 (22. 07. 99) | Date of mailing of the international search report<br>3 August, 1999 (03. 08. 99) |
|---|---|
| Name and mailing address of the ISA/<br>Japanese Patent Office | Authorized officer |
| Facsimile No. | Telephone No. |

Form PCT/ISA/210 (second sheet) (July 1992)